# EUROPÄISCHE PATENTSCHRIFT

(11) **EP 0 919 119 B1**
(45) Veröffentlichungstag und Bekanntmachung des Hinweises auf die Patenterteilung: **25.09.2002**
(21) Anmeldenummer: 98123134.3
(22) Anmeldetag: 07.03.1992
(51) Int. Cl.: A01H 5/00

(54) **Gegen Herbizide von Aryloxy-Phenoxy-Alkancarbonsäuretyp resistenter Mais**
Aryloxy-phenoxy-alkane carboxylic acid herbicide resistant maize
Cellules de mais resistantes aux herbicides du type acide aryloxy-phenoxycarboxylique d'alcane

(30) Priorität: 12.03.1991 EP 91103765
(43) Veröffentlichungstag der Anmeldung: 02.06.1999
(62) Teilanmeldung aus: 92905948.3
(73) Patentinhaber: Bayer CropScience GmbH, 65929 Frankfurt/Main (DE)
(72) Erfinder: Donn, Günter Dr., 65619 Hofheim (DE)

(56) Entgegenhaltungen:
- PARKER, W.B., ET AL.: "Dominant mutations causing alterations in acetyl-coenzyme A carboxylase confer tolerance to cyclohexanedione and aryloxyphenoxypropionate herbicides inmaize" PROCEEDINGS OF THE NATIONAL ACADEMY OF SCIENCES OF USA., Bd. 87, September 1990, Seiten 7175-7179, XP002096800 WASHINGTON US
- DATABASE BIOTECHABS AN 92-04564, WYSE, D.L., ET AL.: "Characterization of cyclohexanedione and aryloxyphenoxyropionte tolearnt maize mutants selected from tissue culture" XP002096803 & LONG ASHTON INT.SYMP.; (1991) 11 MEET., 489-90,
- HOPPE, H.H., ET AL.: "Inhibition of fatty acid biosynthesis in isolated bean and maize chloroplasts by herbicidal phenoxy-phenoxypropionic acid derivatives and structurally related compounds" PESTICIDE BIOCHEMISTRY AND PHYSIOLOGY, Bd. 24, 1985, Seiten 298-305, XP002096801
- CHEMICAL ABSTRACTS, vol. 112, no. 13, 26. März 1990 Columbus, Ohio, US; abstract no. 114108, GRONWALD, J.W., ET AL.: "Selection for tolerance to graminicide herbicides inmaize tissue culture" XP002096802 & BRIGHTON CROP PROT. CONF.--WEEDS, Bd. 3, 1989, Seiten 1217-1224,

## Beschreibung

Herbizide vom Aryloxy-Phenoxy-Alkancarbonsäuretyp (worunter hier auch Heteroaryloxy-Phenoxy-Alkancarbonsäure-Derivate verstanden werden sollen) sind wirksame Gräserherbizide. Stellvertretend für diese Wirkstoffklasse soll im folgenden das Fenoxaprop-ethyl ("FOPE") genannt werden, worunter sowohl das biologisch aktive D-Isomer als auch das Razemat zu verstehen ist. Sie wirken bei Pflanzen der Familie der Poaceae (Gramineae), da nur diese Pflanzenfamilie eine spezielle Form der Acetyl-CoenzymA-carboxylase (ACC) besitzen, die von FOPE in mikromolaren Konzentrationen inhibiert werden kann. Die übrigen Landpflanzen besitzen ACC-Typen, die um den Faktor 100 bis 1000 weniger empfindlich gegenüber dieser Wirkstoffklasse sind.

Da FOPE und andere Herbizide vom Aryloxy-Phenoxy-Alkancarbonsäuretyp über die oberirdischen Pflanzenteile aufgenommen wird, im Boden aber rasch inaktiviert werden, eignen sich diese Herbizide zur Gräserbekämpfung im Nachauflaufverfahren.

Die Kulturpflanze Mais (Zea mays) ist gegenüber FOPE besonders empfindlich. Dies macht den Einsatz dieser Verbindungen zur Bekämpfung von Schadgräsern in Maisfeldern unmöglich.

Es wurde gefunden, daß in Arealen, wo FOPE wiederholt angewendet wurde, in Wildgras-Populationen spontan Formen auftreten, die gegenüber dieser Herbizidklasse resistent sind. Da solche Mutationen nur in einer Rate von etwa 10⁻⁷ bis 10⁻⁹ auftreten, wäre eine entsprechende Suche nach Mutanten in Maisfeldern schon dann wenig aussichtsreich, wenn Mais die besonders hohe Empfindlichkeit gegen FOPE nicht aufwiese.

Unerwarteterweise wurde nun aber gefunden, daß Maiszellen selektiert werden können, die gegen FOPE resistent sind und die zu resistenten Pflanzen kultiviert werden können, die wiederum diese Resistenzeigenschaft stabil weitervererben.

Für die Selektion geeignete Zellinien sind bekannt (beispielsweise aus Morocz et al., Theor. Appl. Genet. 80 (1990) 721-726) bzw. in den Europäischen Patentanmeldungen 90 111 945.3 und 90 111 946.1 vorgeschlagen. Eine geeignete Zellinie wurde mit Wirkung vom 30.09.1990 bei der Deutschen Sammlung von Mikroorganismen und Zellkulturen unter dem Budapester Vertrag mit der Hinterlegungsnummer DSM 6009 hinterlegt.

Um resistente Zellinien zu erhalten, werden die Zellen in auxinfreien Medien in Anwesenheit von FOPE-Konzentrationen kultiviert, die mehr als 90 % der Zellen abtöten. In solchen Medien lassen sich die Zellen beliebig lange kultivieren, beispielsweise ohne weiteres über mehr als 10 Passagen. Synthetische Auxine wie 2,4-Dichlorphenoxyessigsäure, p-Chlorphenoxyessigsäure, 2,4,5-Trichlorphenoxyessigsäure und 3,6-Dichlor-2-methoxybenzoesäure (Dicamba) antagonisieren die Wirkung von FOPE, wenn dieses in subletalen Konzentrationen eingesetzt wird. Selektionsversuche mit FOPE im Konzentrationsbereich bis 10⁻⁵ M in Gegenwart synthetischer Auxine schlugen fehl. Es wurden deshalb auxinautotrophe Maiszellinien eingesetzt.

Auxinautotrophe Zellinien sind dadurch charakterisiert, daß sie beliebig lange, beispielsweise zwei bis drei Jahre, auf phytohormonfreien Zellkulturmedien als embryogene Kultur subkultiviert werden können. Auxinautotrophe Kalli wurden über mehr als 15 Passagen jeweils alle 3 bis 4 Wochen subkultiviert, und durch schrittweise Steigerung der FOPE-Konzentration konnten Mutanten gefunden werden. Diese FOPE-verträglichen Mutanten lassen sich auf FOPE-haltigem auxinfreiem Medium über mehr als 10 Passagen subkultivieren. Unter selektiven Bedingungen, d.h. in Anwesenheit von 10⁻⁴ M FOPE, entwickeln sich aus den embryogenen Kalli spontan Pflanzen, die sich zu fertilen Maispflanzen aufziehen lassen.

Blühende Regeneratpflanzen werden einerseits selbst bestäubt und andererseits Pollen der Regeneratpflanzen zur Bestäubung von Inzuchtlinien eingesetzt. Die reifen Samen werden ausgesät und die Sämlinge der F₁-Generation im 2 bis 4-Blatt-Stadium mit FOPE behandelt. Auch bei Konzentrationen bis zu 200 g Wirkstoff pro ha (g ai/ha) überlebt eine erhebliche Anzahl der Selektanten.

Die regenerierten Maispflanzen können mit bis zu 200 g ai/ha FOPE behandelt werden, vorzugsweise wird zwischen 20 und 150 g, insbesondere zwischen 30 und 90 g ai/ha, eingesetzt. Diese Mengenangaben gelten für das biologisch aktive D-Isomer des Fenoxaprop-ethyls. Erfindungsgemäße Maispflanzen werden vorzugsweise gegen das optisch aktive Isomer selektioniert, es können aber auch entsprechende Mengen des Razemats eingesetzt werden.

Aus den erfindungsgemäßen Mutanten kann in an sich bekannter Weise das ACC-Gen isoliert und charakterisiert werden. Mutierte Gene, die für eine FOPE-verträgliche ACC kodieren, können zur Transformation anderer Pflanzenzellen herangezogen werden.

Weiterhin ist es möglich, die FOPE-Verträglichkeit im Mais mit anderen Herbizid-Resistenzen zu kombinieren. Hierzu setzt man beispielsweise transgene Zellinien ein, die ein Resistenzgen gegen das nicht selektive Herbizid Phosphinothricin, Glufosinat oder Bialaphos enthalten. Derartige Gene sind beispielsweise aus den EP-A 0 257 542, 0 275 957, 0 297 618, aus der DE-A 3 701 623 oder der DE-B 3 825 507 bekannt. Bei der Anzucht entsprechender Zellinien kann die Phosphinothricin-Verträglichkeit als zusätzlicher Marker eingesetzt werden.

Andere erfindungsgemäße transgene Pflanzen können Toxingene, beispielsweise für das Bacillus thuringiensis δ-Endotoxin, oder Gene für Chitinasen oder Glucanasen sowie weitere selektierbare Markergene, beispielsweise Resistenzgene gegen Glyphosate oder Sulfonylharnstoffe enthalten.

Die folgenden Herbizide vom Typ der Aryloxyphenoxy-Alkancarbonsäure-(C₁-C₄)alkyl-, (C₂-C₄)alkenyl- und (C₃-C₄)alkinylester können ebenfalls zur Selektion resistenter Maiszellinien eingesetzt werden:
A1) Phenoxy-phenoxy- und Benzyloxy-phenoxy-Alkancarbonsäure-derivate z. B. 2-(4-(2,4-Dichlorphenoxy)-phenoxy)-propionsäuremethylester (Diclofop-methyl), 2-(4-(4-Brom-2-chlorphenoxy)-phenoxy)-propionsäuremethylester (s. DE-A-2601548),
   2-(4-(4-Brom-2-fluorphenoxy)-phenoxy)-propionsäuremethylester (s. US-A-4808750),
   2-(4-(2-Chlor-4-trifluormethylphenoxy)-phenoxy)-propionsäuremethylester (s. DE-A-2433067),
   2-(4-(2-Fluor-4-trifluormethylphenoxy)-phenoxy)-propionsäuremethylester (s. US-A-4808750),
   2-(4-(2,4-Dichlorbenzyl)-phenoxy)-propionsäuremethylester (s. DE-A-2417487),
   4-(4-(4-Trifluormethylphenoxy)-phenoxy)-pent-2-en-säureethylester,
   2-(4-(4-Trifluormethylphenoxy)-phenoxy)-propionsäuremethylester
   (s. DE-A-2433067),
A2) "Einkernige" Heteroaryloxy-phenoxy-alkancarbonsäurederivate, z. B. 2-(4-(3,5-Dichlorpyridyl-2-oxy)-phenoxy)-propionsäureethylester (s. EP-A-2925), 2-(4-(3,5-Dichlorpyridyl-2-oxy)-phenoxy)-propionsäurepropargylester (EP-A-3114), 2-(4-(3-Chlor-5-trifluormethyl-2-pyridyloxy)-phenoxy-propionsäure-methylester (s. EP-A-3890),
   2-(4-(3-Chlor-5-trifluormethyl-2-pyridyloxy)-phenoxy)-propionsäure-ethylester (s. EP-A-3890),
   2-(4-(5-Chlor-3-fluor-2-pyridyloxy)-phenoxy)-propionsäurepropargylester (EP-A-191736),
   2-(4-(5-Trifluormethyl-2-pyridyloxy)-phenoxy-propionsäurebutylester Fluazifop-butyl (Fusilade-butyl),
A3) "Zweikernige" Heteroaryloxy-phenoxy-alkancarbonsäurederivate, z. B. 2-(4-(6-Chlor-2-chinoxalyloxy)-phenoxy)-propionsäuremethylester und -ethylester (Quizalofop-methyl und -ethyl),
   2-(4-(6-Fluor-2-chinoxalyloxy)-phenoxy)-propionsäuremethylester (s. J. Pest. Sci. Vol. 10, 61 (1985)),
   2-(4-(6-Chlor-2-chinolyloxy)-phenoxy)-propionsäuremethylester und -2-isopropylidenaminooxyethylester (Propaquizafop und Ester), 2-(4-(6-Chlorbenzoxazol-2-yl-oxy)-phenoxy)-propionsäureethylester (Fenoxaprop-ethyl) und
   2-(4-(6-Chlorbenzthiazol-2-yloxy)-phenoxypropionsäureethylester (s. DE-A-2640730)].

Die auxinautotrophen Zellinien eignen sich auch in besonderer Weise für die Selektion von Mutanten, die mit anderen Hemmstoffen der ACC, nämlich den Herbiziden vom Typ der Cyclohexandione, insbesondere Sethoxydim, Tralkoxydim. Cycloxydim, Alloxydim und Clethoxydim erhalten werden.

Aus der Veröffentlichung von Parker et al. (Proc. Natl. Acad. Sci. Vol. 87, pp. 7175-7179) ist bekannt, daß man Maispflanzen erhalten kann, die gegen übliche Konzentrationen von Sethoxydim resistent sind. Außerdem besitzen diese Pflanzen eine gewisse Kreuzresistenz gegen niedrige Konzentrationen von Haloxyfop. Die erfindungsgemäß hergestellten Maispflanzen sind jedoch gegen höhere und für die praktische Anwendung notwendigen Konzentrationen von Aryloxyphenoxyalkancarbon-säureherbiziden resistent. Die erfindungsgemäßen Maispflanzen gestatten somit die selektive Bekämpfung von monokotyledonen Schadpflanzen (Ungräsern) in Mais mittels Aryloxy - (einschließlich Heteroaryloxy)phenoxyalkancarbonsäurederivaten, entweder allein oder in Kombination miteinander.

Die Erfindung betrifft außerdem die Verwendung von Maispflanzen, die mit einer Kombination von Aryloxyphenoxycarbonsäureherbiziden mit Herbiziden gegen dikotyledone Unkräuter behandelt werden. Es konnten nämlich überraschenderweise Kombinationspartner für die Aryloxyphenoxyalkancarbonsäure-herbizide identifiziert werden, die nicht nur von den regenerierten Maispflanzen toleriert werden, sondern deren herbizide Wirkung gleichzeitig verbessert wird.

Die Kombination der Herbizide verursacht also synergistische Effekte. Der Einsatz solcher Mischungen, ist mit großen wirtschaftlichen, aber auch ökologischen Vorteilen verbunden.

Unter Synergismus versteht man die sich gegenseitig verstärkende Wirkung von zwei oder auch mehr Stoffen. Im vorliegenden Fall bewirkt die kombinierte Anwendung von zwei Herbiziden, daß die Aufwandmenge der Herbizide reduziert werden kann und trotzdem die gleiche herbizide Wirkung erreicht wird, bzw. daß mit den gleichen Aufwandmengen der Herbizide eine höhere als die zu erwartende additive Wirkung der einzeln eingesetzten Wirkstoffe erzielt wird.

Durch Nutzung solcher synergistischer Effekte können die Aufwandmengen der beteiligten Mischungspartner erheblich reduziert werden und es gelingt ein breites Spektrum mono- und dikotyler Unkräuter in einem Arbeitsgang zu bekämpfen. Die Reduktion der Aufwandmengen betrifft insbesondere die ACC Hemmstoffe, aber auch die Kombinationspartner bezüglich der Wirksamkeit gegen dikotyle Unkräuter.

Von den Aryloxyphenoxyalkancarbonsäureherbiziden sind die folgenden Herbizide von besonderem Interesse: Fenoxaprop-ethyl, Haloxyfop-methyl, Quizalofop-ethyl, Fluazifop-butyl.

Als Mischungspartner zur zusätzlichen Bekämpfung von breitblättrigen Unkräutern zeigen folgende Herbizide synergistische Wirksamkeit:

Primisulfuron, Thifensulfuron, Nicosulfuron, DPX-E 9636, Amidosulfuron, Pyridylsulfonylharnstoffe, wie in den deutschen Patentanmeldungen P 4000503.8 und P 4030577.5 beschrieben, insbesondere 3-(4,6-Dimethoxypyrimidin-2-yl)-1-[3-(N-methyl-N-methylsulfonyl-amino-2-pyridyl-sulfonyl]-harnstoff, ein Alkoxyphenoxysulfonylharnstoff, wie in EP-A-0342569 beschrieben, ferner NC 319 (EP 282613) und andere Sulfonylharnstoffe sowie Mischungen verschiedener obenerwähnter Sulfonylharnstoffe untereinander wie z. B. eine Mischung aus Nicosulfuron und DPX-E 9636.

Auch andere Herbizide, die den gleichen oder ähnlichen Wirkungsmechanismus haben wie obige Sulfonylharnstoffe, nämlich Imidazolinone, wie z. B. Imazethapyr, Imazaquin, Imazapyr (die jeweils gemeinsam mit einem Safener in Mais eingesetzt werden können), zeigen synergistische Wirkungssteigerung, wenn sie zusammen mit ACC-Inhibitoren eingesetzt werden.

Ebenso kommen hier andere Herbizide in Betracht, die, wie Sulfonylharnstoffe und Imidazolinone, das Enzym Acetolactatsynthase (ALS) hemmen, z. B. substituierte Pyrimidine und Triazine, herbizide Sulfonamide, z. B. Flumetsulam (Cordes, R.C. et al., Abstr. Meet. Weed Sci. Soc. Am. 31, 10, 1991) oder andere verwandte Verbindungen und Mischungen solcher Wirkstoffe untereinander.

Eine Reihe weiterer Herbizide, die zur Bekämpfung von Unkräutern in Mais eingesetzt werden, aber andere Wirkungsmechanismen aufweisen, zeigten ebenfalls synergistische Wirkungssteigerung bei gemeinsamer Anwendung mit Fenoxaprop-ethyl bzw. anderen ACC-Inhibitoren:

ICI-051: (2-[2-chloro-4-(methyl-sulfonyl)benzoyl]-1,3-cyclohexandion, Atrazin, Cyanazin und Terbuthylazin, Clopyralid, Pyridate, Bromoxynil, Pendimethalin, Dicamba.

Die Aufwandmengen der Herbizide liegen im allgemeinen zwischen 0,01 und 2 kg/ha, d. h. die Gesamtmenge an aufzuwendender Wirkstoffkombination beträgt etwa 0,05 bis 2 kg/ha. Die erforderliche Aufwandmenge kann in Abhängigkeit von den äußeren Bedingungen wie Temperatur, Feuchtigkeit u. a. schwanken, vorzugsweise liegt sie zwischen 0,05 und 1 kg/ha. Die Mischungsverhältnisse können innerhalb weiter Grenzen schwanken. Vorzugsweise wird ein Mengenverhältnis zwischen 1:20 und 20:1 gewählt.

Diese synergistische Effekte werden sowohl bei Mischungen mit Fenoxaprop-ethyl wie auch bei Anwendung mit anderen ACC-Hemmstoffen, z. B. den Cyclohexandionen, erzielt. Eine Kombination der Wirkstoffe bedeutet, daß die herbiziden Wirkstoffe gemeinsam ausgebracht oder als sog. split-Applikation einige Tage nacheinander ausgebracht werden. In jedem Falle reagieren die Unkräuter mit erhöhter Sensibilität, so daß mit dem Einsatz geringerer Aufwandmengen eine sehr gute Bekämpfung erzielt werden kann.

In den folgenden Beispielen wird die Erfindung näher erläutert, ohne daß diese darauf beschränkt ist. Prozentangaben beziehen sich auf das Gewicht, sofern keine anderen Angaben gemacht sind.

### 1. Beispiel: Selektion von FOPE-verträglichen embryogenen Maiszellkulturen

Maispflanzen der Inzuchtlinien B 73 und LH 82 wurden mit Pollen des regenerationsfähigen Genotyps HE 89 (Morocz et al., Theor. Appl. Genet. 80 (1990) 721-726 a.a.O.) bestäubt. 12 bis 14 Tage nach der Bestäubung wurden unreife Embryonen unter sterilen Bedingungen aus den Samen herauspräpariert und auf hormonfreien N₆-Kulturmedium (Chu et al., Sci. Sin. 18 (1975) 659-668), das 9 % Saccharose enthält, kultiviert, wobei die Embryoachse dem Medium aufliegt. An ca. 25 % der 1,0 bis 2,0 mm Embryonen bildete sich innerhalb von 3 bis 4 Wochen embryogener Kallus, der auf hormonfreiem Medium subkultivierbar war. Nach 3 bis 4 Subkulturen wurde mit den Kalluslinien, die sich durch gutes Wachstum und reproduzierbare Ausdifferenzierung von somatischen Embryonen auf dem hormonfreien Medium auszeichneten, begonnen, FOPE-verträgliche Mutanten zu selektieren.

Alternativ wurden Kalluslinien über 3 bis 4 Passagen auf N₆-Medium mit 1 mg/l 2,4-Dichlorphenoxyessigsäure (2,4 D) kultiviert. Zur Subkultur wurden die aus undifferenzierten Zellen aufgebauten Kallussektoren herangezogen. Aus diesen Kallussektoren ließen sich Suspensionskulturen induzieren, die in flüssigem N₆-Medium mit 0,5 mg/l 2,4 D kultiviert und wöchentlich in frisches Medium überführt wurden.

Sowohl von den Kalluskulturen als auch von den Suspensionskulturen wurde Gewebe entnommen und auf hormonfreiem N₆-Medium in Anwesenheit von 1-3 x 10⁻⁶ M FOPE 4 bis 6 Wochen inkubiert. Bis zu etwa 95 % der Zellen und Zellcluster starben unter diesen Bedingungen ab.

Die überlebenden Zellcluster wurden auf hormonfreiem N₆-Medium mit 3 x 10⁻⁶ M FOPE über 2 Passen weiter kultiviert. Pro Passage verblieben die Zellen 4 bis 6 Wochen auf dem Selektionsmedium.

Subklone, die unter diesen Bedingungen vergleichbar gut wuchsen wie Wildtypzellen auf FOPE-freiem Medium wurden auf hormonfreies N₆-Medium mit 1 x 10⁻⁵ M FOPE weiterkultiviert.

Nach weiteren 4 bis 6 Wochen wurden diejenigen Klone, die auf diesem Selektionsmedium ohne signifikante Wachstumsreduktion weiterwuchsen, auf ein Medium mit 3 x 10⁻⁵ M FOPE überführt und bei der folgenden Subkultur auf hormonfreies N₆-Medium mit 1 x 10⁻⁴ M Wirkstoff überführt. Höhere Wirkstoffkonzentrationen verstärkten den Selektionseffekt nicht weiter, da der Wirkstoff bereits bei 3 x 10⁻⁵ M im Medium auskristallisiert.

### Beispiel 2

### Regeneration von FOPE-verträglichen Pflanzen

Von denjenigen mutierten Klonen, die in Anwesenheit von 1 x 10⁻⁴ M FOPE in hormonfreiem N₆-Medium über 3 bis 10 Passagen ohne Wachstumsreduktion wuchsen und dabei aus somatischen Embryonen Pflanzen ausdifferenzierten, wurden die Regeneratpflanzen in Erde übergeführt und in einer Klimakammer bei 30 000 bis 40 000 Lux, 14 Stunden Beleuchtung, 23 ± 1 °C Tagestemperatur und 16 ± 1 °C Nachtemperatur aufgezogen. Sobald die Pflanzen 4 bis 5 Blätter entwickelt haben, werden sie mit 30 g FOPE pro ha besprüht. Die Pflanzen überlebten diese Behandlung ohne nennenswerte Schädigung, während Kontrollpflanzen von dieser Herbiziddosis abgetötet wurden.

Die blühenden Regeneratpflanzen wurden einerseits selbstbestäubt und andererseits wurde ihr Pollen dazu verwendet, Inzuchtlinien, wie beispielsweise B 73, LH 51, LH 82, LH 119, KW 1292, KW 5361, RA 1298 oder RA 3080, zu bestäuben. Die reifen Samen wurden ausgesät und die Sämlinge der F₁-Generation im 2 bis 4-Blatt-Stadium mit FOPE behandelt. Die Ergebnisse zeigt die Tabelle 1.

**Tabelle 1**

| Behandlung von Regeneratpflanzen und Nachkommenschaften mit FOPE (∗ unb. = unbeschädigt) | | | | | | | |
|---|---|---|---|---|---|---|---|
| Pflanzen | Anzahl | | | FOPE-Behandlung | | | |
| | | 15 g ai/ha | | 30 g ai/ha | | 60 g ai/ha | |
| | | tote Pfl./unb.∗ | | tote Pfl./unb.∗ | | tote Pfl./unb.∗ | |
| Regeneratpfl. unselektierte Kontrollen | 20 | | | 20 | - | | |
| Regeneratpfl. v. resistenten Kallus | 10 | | | - | 10 | | |
| Selbstungsnachkommen | 60 (3x20) | 5 | 15 | 7 | 13 | 8 | 12 |
| F₁-Kreuzungsnachkommen | 48 (3x16) | 8 | 8 | 10 | 6 | 7 | 9 |
| Handelssorte Felix | 48 | 16 | - | 16 | - | 16 | - |

### 3. Beispiel: Haloxyfop-verträglicher Mais

Nach dem im 1. und 2. Beispiel beschriebenen Verfahren wurden resistente Maiszellinien erhalten und hinsichtlich der Resistenz gegen Haloxyfop untersucht. Dabei stellte sich heraus, daß die erfindungsgemäßen Zellinien eine deutlich höhere Dosis vertragen als die aus dem Stand der Technik bekannten Maiszellinien (siehe Parker et al.).

### 4. Beispiel: Behandlung herbizidresistenter Maispflanzen mit synergistischen Kombinationen von Herbiziden

Nach dem in dem 1. und 2.Beispiel beschriebenen Verfahren erhaltene FOPEresistente Maispflanzen wurden gemeinsam mit grasartigen und breitblättrigen Unkräutern in Töpfen von 9 cm Durchmesser im Gewächshaus bis zum 4 - 6 Blattstadium herangezogen und mit den erfindungsgemäßen Herbiziden im Nachauflauf behandelt. Hierbei wurden ein Wasservolumen von 400 l/ha ausgebracht, 2 Wiederholungen durchgeführt und nach 5 Wochen Bonituren in Form von visuellen Schätzungen der Unkrautbekämpfung nach einer Prozentskala vorgenommen.

Die Ergebnisse der verschiedenen Versuche zeigten unerwartete synergistische Wirkungssteigerungen durch die gemeinsam oder kurz hintereinander ausgebrachten Herbizid-Kombinationen (siehe Tabelle 2 und 3).

An den herbizidresistenten Maispflanzen wurden keinerlei Schädigungen beobachtet. Die Herbizide B4 und B6 wurde jeweils gemeinsam mit einem Safenerwirkstoff appliziert.

**Tabelle 2:**

| Herbizide Wirksamkeit gegen Gräser | | | | | | | |
|---|---|---|---|---|---|---|---|
| Herbizid | Dosis | % Wirkung gegen | | | | | |
| | g AS/ha | SEVI | DISA | PAMI | ECCG | SOHA | ZEMA |
| A | 50 | 100 | 100 | - | - | - | 0 |
| | 25 | 100 | 99 | 100 | 100 | 98 | 0 |
| | 12 | 85 | 85 | 100 | 100 | 85 | 0 |
| | 6 | 60 | 50 | 80 | 99 | 40 | 0 |
| B1 | 50 | 98 | 93 | 65 | 98 | 60 | 0 |
| | 25 | 95 | 85 | 20 | 95 | 35 | 0 |
| | 12 | 85 | 75 | 10 | 80 | 25 | 0 |
| B2 | 12 | 0 | 10 | 0 | 0 | 20 | 0 |
| | 6 | 0 | 5 | 0 | 0 | 0 | 0 |
| B3 | 50 | 50 | 20 | 30 | 30 | 90 | 0 |
| | 25 | 40 | 10 | 0 | 20 | 80 | 0 |
| | 12 | 30 | 0 | 0 | 20 | 70 | 0 |
| B4 | 25 | 80 | 90 | 65 | 95 | 45 | 0 |
| | 12 | 70 | 85 | 40 | 90 | 30 | 0 |
| | 6 | 60 | 70 | 15 | 75 | 20 | 0 |
| B5 | 25 | 85 | 95 | 70 | 90 | 70 | 0 |
| | 12 | 80 | 90 | 40 | 80 | 50 | 0 |
| | 6 | 80 | 80 | 25 | 70 | 30 | 0 |
| B6 | 100 | 95 | 90 | 70 | 70 | 60 | 0 |
| | 50 | 80 | 80 | 60 | 70 | 40 | 0 |
| B7 | 250 | 5 | 0 | 0 | 10 | 0 | 0 |
| | 125 | 0 | 0 | 0 | 0 | 0 | 0 |
| B8 | 250 | 0 | 0 | 0 | 0 | 0 | 0 |
| | 125 | 0 | 0 | 0 | 0 | 0 | 0 |
| B9 | 250 | 0 | 0 | 0 | 5 | 0 | 0 |
| | 125 | 0 | 0 | 0 | 0 | 0 | 0 |
| A + B1 | 12 + 12 | 100 | 100 | 100 | 100 | 98 | 0 |
| | 12 + 25 | 100 | 98 | 95 | 100 | 80 | 0 |
| A + B2 | 12 + 12 | 100 | 100 | 100 | 100 | 99 | 0 |
| | 6 + 12 | 100 | 99 | 100 | 100 | 75 | 0 |
| A + B3 | 12 + 12 | 100 | 100 | 100 | 100 | 100 | 0 |
| | 12 + 25 | 100 | 100 | 100 | 100 | 100 | 0 |
| A + B4 | 12 + 12 | 100 | 100 | 100 | 100 | 99 | 0 |
| | 6 + 6 | 100 | 95 | 95 | 100 | 90 | 0 |
| A + B5 | 12 + 12 | 100 | 100 | 100 | 100 | 100 | 0 |
| | 6 + 6 | 100 | 98 | 98 | 100 | 100 | 0 |
| A + B6 | 12 + 50 | 100 | 100 | 100 | 100 | 100 | 0 |
| | 6 + 25 | 100 | 99 | 98 | 100 | 95 | 0 |
| A + B7 | 12 +125 | 95 | 90 | 100 | 100 | 95 | 0 |
| A + B8 | 12 +125 | 90 | 95 | 100 | 99 | 90 | 0 |
| A + B9 | 12 +125 | 95 | 90 | 99 | 99 | 90 | 0 |

**Tabelle 3:**

| Herbizide Wirksamkeit gegen Breitblättrige | | | | | | | |
|---|---|---|---|---|---|---|---|
| Herbizid | Dosis g AS/ha | ABTH | CHA L | AMAR | POCO | AMRE | ZEMA |
| A | 50 | 0 | 0 | 0 | 0 | 5 | 0 |
| | 25 | 0 | 0 | 0 | 0 | 0 | 0 |
| | 12 | 0 | 0 | 0 | 0 | 0 | 0 |
| | 6 | 0 | 0 | 0 | 0 | 0 | 0 |
| B1 | 50 | 20 | 35 | 20 | 50 | 60 | 0 |
| | 25 | 10 | 20 | 10 | 30 | 50 | 0 |
| | 12 | 0 | 10 | 0 | 10 | 50 | 0 |
| B2 | 12 | 40 | 80 | 70 | 80 | 20 | 0 |
| | 6 | 20 | 60 | 40 | 50 | 10 | 0 |
| B3 | 50 | 70 | 80 | 30 | 40 | 40 | 0 |
| | 25 | 50 | 70 | 10 | 20 | 30 | 0 |
| | 12 | 40 | 50 | 0 | 20 | 15 | 0 |
| B4 | 25 | 20 | 95 | 85 | 80 | 80 | 0 |
| | 12 | 10 | 85 | 80 | 70 | 75 | 0 |
| | 6 | 0 | 50 | 70 | 70 | 60 | 0 |
| B5 | 25 | 30 | 80 | 75 | 30 | 60 | 0 |
| | 12 | 20 | 60 | 60 | 20 | 50 | 0 |
| | 6 | 10 | 40 | 40 | 10 | 30 | 0 |
| B6 | 100 | 60 | 40 | 70 | 50 | 80 | 0 |
| | 50 | 50 | 30 | 60 | 40 | 70 | 0 |
| B7 | 250 | 40 | 100 | 75 | 80 | 75 | 0 |
| | 125 | 15 | 85 | 40 | 50 | 30 | 0 |
| B8 | 250 | 70 | 95 | 70 | 85 | 65 | 0 |
| | 125 | 30 | 75 | 30 | 60 | 30 | 0 |
| B9 | 250 | 90 | 100 | 98 | 80 | 75 | 0 |
| | 125 | 75 | 80 | 70 | 40 | 60 | 0 |
| A+B1 | 12 + 12 | 40 | 50 | 30 | 60 | 90 | 0 |
| | 12 + 25 | 40 | 30 | 30 | 40 | 75 | 0 |
| A+B2 | 12 + 12 | 70 | 90 | 80 | 95 | 40 | 0 |
| | 6 + 12 | 60 | 80 | 75 | 90 | 40 | 0 |
| A+B3 | 12 + 12 | 60 | 70 | 30 | 50 | 30 | 0 |
| | 12 + 25 | 70 | 80 | 40 | 50 | 40 | 0 |
| A + B4 | 12 + 12 | 50 | 95 | 90 | 90 | 80 | 0 |
| | 6 + 6 | 30 | 60 | 80 | 90 | 75 | 0 |
| A + B5 | 12 + 12 | 50 | 90 | 95 | 40 | 90 | 0 |
| | 6 + 6 | 40 | 75 | 50 | 30 | 60 | 0 |
| A + B6 | 12 + 50 | 70 | 50 | 95 | 70 | 80 | 0 |
| | 6 + 25 | 50 | 30 | 60 | 40 | 60 | 0 |
| A + B7 | 12 +125 | 40 | 90 | 50 | 70 | 50 | 0 |
| A + B8 | 12 +125 | 60 | 80 | 40 | 60 | 40 | 0 |
| A + B9 | 12 +125 | 90 | 90 | 80 | 50 | 60 | 0 |

### Legende zu Tabelle 2 und 3:

- SEVI =: Setaria viridis (Grüne Borstenhirse)
- DISA =: Digitaria sanguinalis (Bluthirse)
- PAMI =: Panicum miliaceum (Futterhirse)
- ECCG =: Echinochloa crus galli (Hühnerhirse)
- SOHA =: Sorghum halepense (Johnsongras)
- ABTH =: Abutilon theophrasti (Samtpappel)
- CHAL =: Chenopodium album (Weißer Gänsefuß)
- AMAR =: Ambrosia artemisifolia (Hohe Ambrosia)
- POCO =: Polygonum convolvulus (Windknöterich)
- AMRE =: Amaranthus retroflexus (Amaranth)
- ZEMA =: Zea mays (Mais)

- A =: Fenoxaprop-p-ethyl
- B1 =: Nicosulfuron
- B2 =: Thifensulfuron
- B3 =: Primisulfuron
- B4 =: 3-(4,6-Dimethoxypyrimidin-2-yl)-1-[3-(N-methyl-N-methylsulfonyl-amino)-2-pyridyl-sulfonyl]-harnstoff
- B5 =: DPX-9636
- B6 =: Imazethapyr
- B7 =: Atrazin
- B8 =: Bromoxynil
- B9 =: Dicamba

## Patentansprüche

1. Maispflanzen, die gegenüber den in der Unkrautkontrolle üblichen Anwendungskonzentrationen von Aryloxyphenoxyalkancarbonsäureherbiziden beständig sind, sowie deren Samen.

2. Maispflanzen gemäß Anspruch 1, **dadurch gekennzeichnet, daß** sie aus auxinautotrophen Maiszellen, Zellkulturen und Kalli regeneriert sind, und deren Samen und Nachkommenschaften.

3. Pflanzen nach einem oder mehreren der Ansprüche 1 und 2, die eine Resistenz gegen ein weiteres Herbizid aufweisen.

4. Maispflanzen gemäß einem oder mehreren der Ansprüche 1 bis 3, **dadurch gekennzeichnet, daß** Fenoxaprop-ethyl als Herbizid eingesetzt wird.

5. Maispflanzen gemäß einem oder mehreren der Ansprüche 1 bis 3, die gegenüber Aufwandmengen zwischen 20 und 200 g Fenoxaprop-ethyl ai/ha resistent sind.

6. Maispflanzen, gemäß einem oder mehreren der Ansprüche 1 bis 3, die gegenüber Aufwandmengen zwischen 30 und 150 g Fenoxaprop-ethyl ai/ha resistent sind.

7. Maispflanzen gemäß einem oder mehreren der Ansprüche 1 bis 3, die gegenüber Aufwandmengen zwischen 30 und 90 g Fenoxaprop-ethyl ai/ha resistent sind.

8. Maispflanzen gemäß einem oder mehreren der Ansprüche 1 bis 3, die gegenüber Aryloxyphenoxyalkancarbonsäureherbizide und Glutaminsynthetaseinhibitoren resistent sind.

9. Maispflanzen gemäß einem oder mehreren der Ansprüche 1 bis 3, die gegenüber Fenoxaprop-ethyl und Phosphinothricin oder Bialapos resistent sind.

10. Verwendung von Aryloxy-( inklusive Heteroaryloxy-)phenoxyalkancarbonsäureherbiziden, allein oder in Kombination miteinander, zur Gräserbekämpfung in Kulturen von Maispflanzen gemäß einem oder mehreren der Ansprüche 1 bis 3

11. Verwendung von Aryloxyphenoxyalkancarbonsäureherbiziden in Kombination mit einem oder mehreren Herbiziden ausgewählt aus der Gruppe der Sulfonylharnstoffe und Imidazoline zur Bekämpfung von mono- und dicotyledonen Unkräutern in Kulturen von Maispflanzen gemäß einem oder mehreren der Ansprüche 1 bis 3.

12. Verwendung von Fenoxaprop-ethyl in Kombination mit Sulfonylharnstoffen zur Bekämpfung von mono- und dicotyledonen Unkräutern in Kulturen von Maispflanzen gemäß einem oder mehreren der Ansprüche 1-3

13. Verwendung von Fenoxaprop-ethyl in Kombination mit einem oder mehreren Herbiziden aus der Gruppe der Triazine (Atrazin, Cyanazin, Terbutylazin) Clopyralid, Pyridate, Bromoxynil, Pendimethalin oder Dicamba zur Bekämpfung von mono- und dicotyledonen Unkräutern in Kulturen von Maispflanzen gemäß einem der Ansprüche 1 bis 3.

14. Verfahren zur Gräserbekämpfung in Maiskulturen, **dadurch gekennzeichnet, daß** die Kulturen aus Maispflanzen gemäß einem oder mehreren der Ansprüche 1 bis 3 mit einem oder mehreren Herbiziden aus der Gruppe der Aryloxyphenoxyalkancarbonsäureherbiziden in zur Unkrautkontrolle üblichen Anwendungskonzentrationen behandelt werden.

## Claims

1. A maize plant which is resistant to aryloxyphenoxyalkanecarboxylic acid herbicides, and seeds thereof, in use concentrations customary in the control of weeds.

2. A maize plant as claimed in claim 1 which is regenerated from auxin-autotrophic maize cells, cell cultures and calli, and seeds and progeny thereof.

3. A plant as claimed in one or both of claims 1 and 2 which is resistant to a further herbicide.

4. A maize plant as claimed in one or more of claims 1 to 3, wherein the herbicide used is fenoxapropethyl.

5. A maize plant as claimed in one or more of claims 1 to 3, which is resistant to fenoxaprop-ethyl at application rates of between 20 and 200 g of a.i./ha.

6. A maize plant as claimed in one or more of claims 1 to 3, which is resistant to fenoxaprop-ethyl at application rates of between 30 and 150 g of a.i./ha.

7. A maize plant as claimed in one or more of claims 1 to 3, which is resistant to fenoxaprop-ethyl at application rates of between 30 and 90 g of a.i./ha.

8. A maize plant as claimed in one or more of claims 1 to 3, which is resistant to aryloxyphenoxyalkanecarboxylic acid herbicides and glutamine synthetase inhibitors.

9. A maize plant as claimed in one or more of claims 1 to 3, which is resistant to fenoxaprop-ethyl and phosphinothricin or bialaphos.

10. The use of aryloxyphenoxyalkanecarboxylic (including heteroaryloxyphenoxyalkanecarboxylic) acid herbicides, alone or in combination with each other, for controlling grasses in cultures of maize plants as claimed in one or more of claims 1 to 3.

11. The use of aryloxyphenoxyalkanecarboxylic acid herbicides in combination with one or more herbicides selected from the group of the sulfonylureas and imidazolines for controlling mono- and dicotyledon weeds in cultures of maize plants as claimed in one or more of claims 1 to 3.

12. The use of fenoxaprop-ethyl in combination with sulfonylureas for controlling mono- and dicotyledon weeds in cultures of maize plants as claimed in one or more of claims 1 to 3.

13. The use of fenoxaprop-ethyl in combination with one or more herbicides from the group of the triazines (atrazine, cyanazine, terbutylazine), clopyralid, pyridate, bromoxynil, penaimethalin or dicamba for controlling mono- and dicotyledon weeds in cultures of maize plants as claimed in one of claims 1 to 3.

14. A method for controlling grasses in cultures of maize plants as claimed in one or more of claims 1 to 3 using one or more herbicides from the group of the aryloxyphenoxyalkanecarboxylic acid herbicides in use concentrations customary for the control of weeds.

## Revendications

1. Plants de maïs qui sont résistants aux herbicides du type acide aryloxy-phenoxycarboxylique d'alcane aux concentrations d'usage courantes dans le contrôle des mauvaises herbes, ainsi que leurs semences.

2. Plants de maïs selon la revendication 1, **caractérisés en ce qu'**ils sont régénérés de cellules de maïs auxine-autotrophes , de cultures de cellules et de cals, et leurs semences et leurs descendants.

3. Plants selon l'une ou plusieurs des revendications 1 et 2, qui montrent une résistance à un autre herbicide.

4. Plants de maïs selon une ou plusieurs des revendications 1 à 3, **caractérisés en ce que** le fenoxapropethyl est utilisé en tant qu'herbicide.

5. Plants de maïs selon une ou plusieurs des revendications 1 à 3, qui sont résistants à des quantités entre 20 et 200 g de fenoxapropethyl a.i./ha.

6. Plants de maïs selon une ou plusieurs des revendications 1 à 3, qui sont résistants à des quantités entre 30 et 150 g de fenoxapropethyl a.i./ha.

7. Plants de maïs selon une ou plusieurs des revendications 1 à 3, qui sont résistants à des quantités entre 30 et 90 g de fenoxapropethyl a.i./ha.

8. Plants de maïs selon une ou plusieurs des revendications 1 à 3, qui sont résistants aux herbicides du type acide aryloxy-phenoxycarboxylique d'alcane et aux inhibiteurs de glutamine synthase.

9. Plants de maïs selon une ou plusieurs des revendications 1 à 3, qui sont résistants au fenoxapropethyl et à la phosphinothricine ou au bialaphos.

10. Utilisation des herbicides du type acide aryloxy-( y compris heteroaryloxy-) phenoxycarboxylique d'alcane seuls ou en combinaison ensemble, pour la lutte contre les herbes dans des cultures de plants de maïs selon une ou plusieurs des revendications 1 à 3.

11. Utilisation des herbicides du type acide aryloxy phenoxycarboxylique d'alcane en combinaison avec un ou plusieurs herbicides choisis parmi le groupe des sulfonylurées et des imidazolines pour la lutte contre les mauvaises herbes mono- et dicotyledones dans des cultures de plants de maïs selon une ou plusieurs des revendications 1 à 3

12. Utilisation du fenoxapropethyl en combinaison avec des sulfonylurées pour la lutte contre les mauvaises herbes mono- et dicotyledones dans des cultures de plants de maïs selon une ou plusieurs des revendications 1 à 3

13. Utilisation du fenoxapropethyl en combinaison avec un ou plusieurs herbicides du groupe des triazines (atrazine, cyanazine, terbutylazine) clopyralid, pyridate, bromoxynil, pendimethalin ou dicamba pour la lutte contre les mauvaises herbes mono- et dicotyledones dans des cultures de plants de maïs selon une des revendications 1 à 3

14. Procédé de lutte contre les herbes dans les cultures de maïs, **caractérisé en ce que** les cultures des plants de maïs selon une ou plusieurs des revendications 1 à 3 sont traitées avec un ou plusieurs herbicides du groupe des herbicides du type acide aryloxy-phenoxycarboxylique d'alcane à des concentrations d'usage courantes dans le contrôle des mauvaises herbes
